# EUROPEAN PATENT APPLICATION

(11) **EP 4 643 920 A1**
(43) Date of publication of application: **05.11.2025**
(21) Application number: 23924140.9
(22) Date of filing: 25.08.2023
(51) Int. Cl.: A61M 37/00

(54) **MICRONEEDLE PATCH**

(30) Priority: 20.02.2023 JP 2023024291
(71) Applicant: Hisamitsu Pharmaceutical Co., Inc., Tosu-shi, Saga 841-0017 (JP)
(72) Inventor: KOIZUMI, Nao, Tsukuba-shi, Ibaraki 305-0856 (JP); TAKEUCHI, Akio, Tsukuba-shi, Ibaraki 305-0856 (JP); NISHIMURA, Shinpei, Tsukuba-shi, Ibaraki 305-0856 (JP)
(74) Representative: Grünecker Patent- und Rechtsanwälte PartG mbB
(86) International application number: PCT/JP2023/030778
(87) International publication number: WO 2024/176495

(57) **Abstract**

A microneedle patch, includes: a backing; an adhesive layer provided on a main surface of the backing; and a microneedle array, at least part of the microneedle array being positioned within the adhesive layer. The adhesive layer includes a water-soluble polymer, glycerin, and water. A content of the water is 45 to 70% by mass with respect to a total mass of the adhesive layer. The microneedle array includes a base and at least one microneedle standing from a main surface of the base. The adhesive layer includes a main portion positioned on the microneedle array. A thickness of the main portion is 0.9 to 1.3 times a length of the microneedle.

## Description

### Technical Field

One aspect of the present disclosure relates to a microneedle patch.

### Background Art

In Patent Literature 1, a patch with fine needles is described. This patch includes one or more fine needle members capable of protruding from an adhesive layer to the sticking side, in the adhesive layer.

In Patent Literature 2, a patch is described that includes a backing, an adhesive layer provided on one surface of the backing and including an active ingredient, and a microneedle array having microneedles provided on a base material. After the microneedles are exposed from the inside of the adhesive layer and stuck into the skin, they move in a direction away from the skin due to elasticity.

### Citation List

### Patent Literature

Patent Literature 1: JP 2007-1938 A
Patent Literature 2: JP 2015-151380 A

### Summary of Invention

### Technical Problem

A microneedle patch is demanded that is capable of efficiently performing transdermal administration of an active ingredient.

### Solution to Problem

A microneedle patch according to one aspect of the present disclosure includes: a backing; an adhesive layer provided on a main surface of the backing; and a microneedle array, at least part of the microneedle array being positioned within the adhesive layer, wherein the adhesive layer includes a water-soluble polymer, glycerin, and water, a content of the water is 45 to 70% by mass with respect to a total mass of the adhesive layer, the microneedle array includes a base and at least one microneedle standing from a main surface of the base, the adhesive layer includes a main portion positioned on the microneedle array, and a thickness of the main portion is 0.9 to 1.3 times a length of the microneedle.

In such an aspect, each of the water content and the ratio of the thickness of the adhesive layer on the microneedle array to the length of the microneedle is set within the corresponding numerical range described above. With this configuration, while adhesion of the microneedle patch to the skin is maintained, the skin is stretched by the microneedle protruding from the adhesive layer. As a result, the contact area between the stretched skin and the adhesive layer increases, so that transdermal administration of the active ingredient may be efficiently performed.

### Advantageous Effects of Invention

According to one aspect of the present disclosure, transdermal administration of an active ingredient may be efficiently performed.

### Brief Description of Drawings

[Figure 1] Figure 1 is a perspective view showing one example of a microneedle patch.
[Figure 2] Figure 2 is an enlarged perspective view showing one example of a microneedle array.
[Figure 3] Figure 3 is a diagram schematically showing the microneedle patch viewed from the side.
[Figure 4] Figure 4 is a diagram schematically showing changes in the microneedle patch before and after application to skin.
[Figure 5] Figure 5 is a diagram schematically showing application of the microneedle patch to skin.

### Description of Embodiments

Hereinafter, the embodiments of the present disclosure will be described in detail with reference to the attached drawings. In the drawings, the same or equivalent elements are designated by the same reference numerals, and repeated descriptions will be omitted.

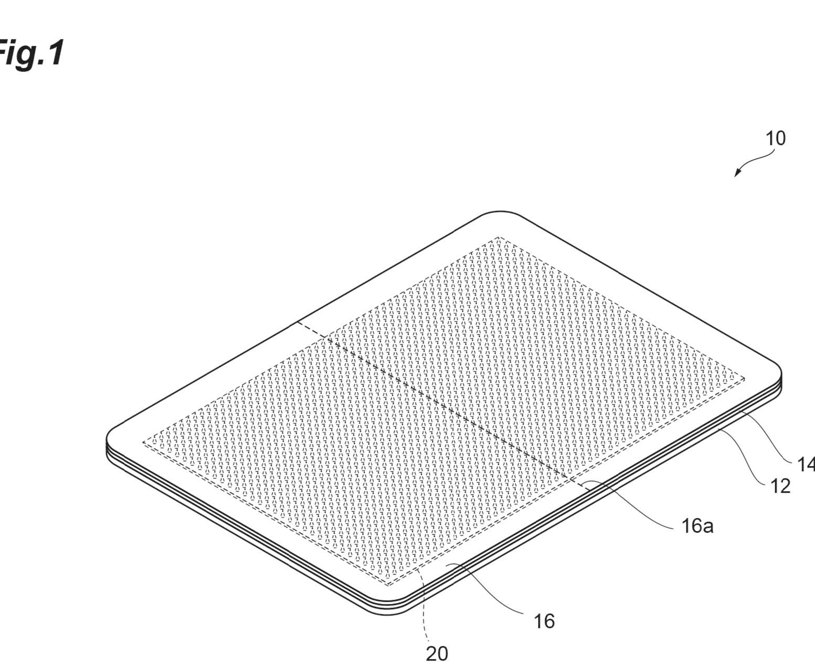

Figure 1 is a perspective view showing a microneedle patch 10 according to one example. The microneedle patch 10 is a formulation used as a gel patch, a tape, or the like. In one example, the microneedle patch 10 includes a backing 12, an adhesive layer 14 formed on the substantially entire main surface of the backing 12, a release sheet 16 releasably affixed to the action surface of the adhesive layer 14, and a microneedle array 20 embedded in the adhesive layer 14. The action surface of the adhesive layer 14 refers to a surface being in contact with skin of a user upon application of the microneedle patch 10.

The area of the microneedle patch 10 may be set depending on the symptom, age, body weight, sex, and the like of the user. For example, the area of the microneedle patch 10 may be 1 to 500 cm² or may be 10 to 400 cm². By setting the area to 1 cm² or more, it becomes easy to maintain the sufficient skin permeability of an active ingredient. By setting the area to 500 cm² or less, the handling of the microneedle patch 10 becomes easy.

The backing 12 is a sheet-shaped member. The backing 12 may have stretchability. The material of the backing 12 may be selected in consideration of physical properties (such as thickness, elongation, tensile strength, and affixing workability), feeling at the time of affixing, sealing properties of the skin, transfer of an active ingredient to the backing 12, and the like. For example, the material may be a woven fabric, a non-woven fabric, a resin film, a foamed sheet, or paper. Examples of the woven fabric may include a knitted fabric. In a case where a woven fabric, a non-woven fabric, or a resin film is used as the backing 12, examples of components thereof include polyolefins such as polyethylene, polypropylene, and polybutylene, polyesters such as polyethylene terephthalate (PET), rayon, polyurethane, and cotton. One of these components may be used alone, or two or more may be used in combination. The backing 12 may have a single-layer structure or a multilayer structure. The backing 12 may be a sheet-shaped porous material or a sheet-shaped foam.

Examples of the knitted fabric include a knitted fabric obtained from polyester, nylon, polypropylene, rayon material, or from any combination thereof. For example, a knitted fabric consisting of polyethylene terephthalate, which is a polyester material and in which the interaction with the active ingredient is small, may be used.

In a case where the backing 12 is a knitted fabric or a non-woven fabric, spreading a water-containing adhesive composition onto the backing 12 may cause the adhesive composition to ooze out to the back side of the backing through the meshes of the backing 12. In consideration of this matter, the basis weight of the knitted fabric or non-woven fabric may be 50 to 150 g/m², or may be 75 to 125 g/m². By setting the basis weight to such a range, there is a tendency that the adhesive composition can be spread without allowing it to ooze out to the back side of the backing 12 through the gaps in the backing 12. In addition, it is possible to maintain anchoring properties between the backing 12 and the adhesive layer 14.

As for the stretchability of the backing 12, the 50% modulus (load at 50% elongation) may be 0.5 to 10 N/50 mm in at least one of the vertical direction and the horizontal direction. Here, the term "vertical direction" refers to a flowing direction in the process of producing a knitted fabric, and the term "horizontal direction" refers to a direction orthogonal to the vertical direction, that is, the width direction. The method for measuring the modulus is based on JIS L 1018:1999.

The thickness of the backing 12 may be set within a range of, for example, 5 to 1000 µm. Setting the thickness to 5 µm or more is to make the handling of the microneedle patch 10 easy. Setting the thickness to 1000 µm or less is to prevent the influence of the hardness of the backing 12 on the adhesion of the microneedle patch 10.

The adhesive layer 14 is a layer having tackiness that can adhere to the skin of a human. In one example, the adhesive layer 14 includes a water-soluble polymer and water. The adhesive layer 14 may further include an active ingredient (physiologically active substance). The term "the adhesive layer includes an active ingredient" refers to a concept including both an aspect in which an active ingredient is present in the adhesive layer 14, and an aspect in which an active ingredient is attached to the action surface of the adhesive layer 14.

The water-soluble polymer means a macromolecule having a hydrophilic group. Examples of the hydrophilic group include a hydroxy group, a carboxy group, and an amino group. Due to the water-soluble polymer contained in the adhesive layer 14, water in the microneedle patch 10 can be retained for a longer period of time.

The water-soluble polymer may be a neutralized polyacrylate (these may be referred to as "water-soluble acrylic polymer"). Due to the adhesive layer 14 containing the neutralized polyacrylate, the adhesive layer 14 with better adhesion may be provided.

The water-soluble acrylic polymer is a polymer obtained by polymerizing an acryloyl group-containing compound having a functional group (a hydrophilic group) that exerts water solubility. The adhesive layer 14 exerts adhesion by containing a water-soluble acrylic polymer with water. The water-soluble acrylic polymer is a polymer obtained by, for example, polymerizing a compound having an acryloyl group, such as a polyacrylic acid or neutralized product thereof, an acrylic acid ester having a hydrophilic group, or an acrylic acid amide having a hydrophilic group. The water-soluble acrylic polymer may be a homopolymer obtained from one compound having an acryloyl group, or may be a copolymer obtained from two or more compounds having acryloyl groups.

The hydrophilic group may be any of the cationic hydrophilic group, anionic hydrophilic group, and nonionic hydrophilic group. Examples of the cationic hydrophilic group include a quaternary ammonium group. Examples of the anionic hydrophilic group include a carboxy group, a sulfo group, and a phosphoric acid group. Examples of the nonionic hydrophilic group include a hydroxy group and an amino group.

The neutralized polyacrylate may be a completely-neutralized polyacrylate, a partially-neutralized polyacrylate, or a mixture thereof. Further, polyacrylic acid may be mixed. From the viewpoint of adjusting the pH of the adhesive layer to easily suppress decomposition of components contained in the adhesive layer, the neutralized polyacrylate may be a mixture of a partially-neutralized polyacrylate and polyacrylic acid. Examples of the completely-neutralized polyacrylate and the partially-neutralized polyacrylate include polyacrylic acid salts, and for example, a sodium salt, a potassium salt, a calcium salt, and an ammonium salt can be used. In a case where a neutralized polyacrylate is contained in the adhesive layer 14 as a water-soluble polymer, the content thereof may be regulated to 1 to 15% by mass, 2 to 12% by mass, or 5 to 10% by mass, with respect to the total mass of the adhesive layer 14. With the content of the neutralized polyacrylate being 1% by mass or more, sufficient adhesion strength of the neutralized polyacrylate can be obtained. With the content of the neutralized polyacrylate being 12% by mass or less, the moldability and shape retainability of the adhesive layer 14 are improved.

As the neutralized polyacrylate, a partially-neutralized polyacrylate whose initial adhesion strength and over-time adhesion strength are high may be used. The partially-neutralized polyacrylate is a composition having a structural unit derived from acrylic acid and a structural unit derived from an acrylic acid salt in any proportion in one polymer chain. A partially-neutralized polyacrylate in which 20 to 80 mol% of the carboxy groups in one polymer chain are neutralized may be used.

In a case where at least one of a completely-neutralized polyacrylate and a partially-neutralized polyacrylate is contained in the adhesive layer 14 as the water-soluble polymer, the total content thereof may be regulated to 1 to 10% by mass or 2 to 9% by mass, with respect to the total mass of the adhesive layer 14. With the total content of at least one of the completely-neutralized polyacrylate and the partially-neutralized polyacrylate being 1% by mass or more, sufficient adhesion strength of the adhesive layer 14 can be obtained. With the total content of at least one of the completely-neutralized polyacrylate and the partially-neutralized polyacrylate being 9% by mass or less, the moldability and shape retainability of the adhesive layer 14 are improved.

In a case where polyacrylic acid is further contained in the adhesive layer 14 as the water-soluble polymer, the content thereof may be regulated to 0.1 to 5% by mass, 0.2 to 4% by mass, or 0.3 to 3% by mass, with respect to the total mass of the adhesive layer 14. With the content of the polyacrylic acid being 0.1% by mass or more, there is a tendency that the moldability and shape retainability of the adhesive layer 14 are more improved. With the content of the polyacrylic acid being 5% by mass or less, there is a tendency that the hardness of the adhesive layer 14 is unlikely to be high and adhesion to the skin is more increased.

In the acrylic acid ester having a hydrophilic group, the acrylic acid ester moiety may be an alkyl acrylate. The alkyl moiety may be an alkyl having 1 to 10 carbon atoms or an alkyl having 1 to 8 carbon atoms. In the acrylic ester having a hydrophilic group, the hydrophilic group may be present in that alkyl moiety.

An ingredient other than the water-soluble acrylic polymer may be contained in the adhesive layer 14 as a water-soluble polymer. For example, the adhesive layer 14 may contain gelatin, polyvinyl alcohol, polyvinylpyrrolidone, sodium alginate, sodium hyaluronate, hydroxypropyl cellulose, sodium carboxymethylcellulose (carmellose sodium), methylcellulose, carrageenan, glucomannan, agar, guar gum, xanthan gum, gellan gum, pectin, or locust bean gum. One of these may be used singly, or two or more may be used in combination. For example, carmellose sodium, gelatin, or polyvinyl alcohol may be used. These ingredients may be used in combination with the water-soluble acrylic polymer.

In a case where the adhesive layer 14 contains a water-soluble polymer other than the water-soluble acrylic polymer, the content thereof may be regulated to 0.1 to 30% by mass, 3 to 18% by mass, or 3 to 10% by mass, with respect to the total mass of the adhesive layer 14. With the content of the water-soluble polymer other than the water-soluble acrylic polymer being 3% by mass or more, there is a tendency that the cohesive strength of the adhesive layer 14 is likely to be high. With the content being 10% by mass or less, there is a tendency that the active ingredient contained in the adhesive layer 14 is likely to be uniformly dispersed.

Due to the adhesive layer 14 containing water, the skin permeability of the active ingredient is improved and the action of the active ingredient is more effectively exerted. The content of the water may be 45 to 70% by mass with respect to the total mass of the adhesive layer 14. With the content of water being 70% by mass or less, there is a tendency that the moldability and shape retainability of the adhesive layer are more increased.

The adhesive layer 14 may contain a methyl acrylate/2-ethylhexyl acrylate copolymer resin. In a conventional patch, when the mass of the adhesive layer 14 is small, the water content is likely to be low and adhesion strength is likely to be poor. Due to the methyl acrylate/2-ethylhexyl acrylate copolymer resin contained in the adhesive layer 14, there is a tendency that a sufficient adhesion strength is likely to be maintained even after a lapse of a long period of time even in a case where the mass of the adhesive layer 14 is relatively small.

The adhesive layer 14 may contain a solubilizer, a crosslinking agent, a moisturizer, a cooling agent, a stabilizer, a filler, a preservative, a chelating agent, an inorganic powder, a colorant, a flavoring, or a pH adjuster as a further ingredient.

The solubilizer is used to dissolve the active ingredient. Examples of the solubilizer include crotamiton; N-methylpyrrolidone; polyalkylene glycols such as polyethylene glycol (PEG) and polybutylene glycol; fatty acid esters such as isopropyl myristate and diethyl adipate; oxyalkylene fatty acid esters such as polyethylene glycol monostearate; fatty acid esters such as polyoxyalkylene sorbitan fatty acid esters; polyoxyethylene hydrogenated castor oil; and surfactants such as polysorbate 80. One of these solubilizers may be used singly, or two or more may be used in combination. The content of the solubilizer may be regulated to 0.1 to 10% by mass with respect to the total mass of the adhesive layer 14.

The crosslinking agent is used to make the adhesive layer 14 robust and have water retention properties. Examples of the crosslinking agent include thermosetting resins such as amino resins, phenol resins, epoxy resins, alkyd resins, and unsaturated polyesters, isocyanate compounds, block isocyanate compounds, organic crosslinking agents, potassium aluminum sulfate (alum), aluminum silicate, magnesium sulfate, magnesium aluminum metasilicate, dihydroxyaluminum aminoacetate, aluminum hydroxide gel, and inorganic crosslinking agents such as metals or metal compounds. The content of the crosslinking agent may be 0.01 to 10% by mass, 0.01 to 6.5% by mass, 0.01 to 3% by mass, 0.05 to 2% by mass, or 0.1 to 1% by mass, with respect to the total mass of the adhesive layer 14.

The moisturizer is used to suppress the evaporation of water from the adhesive layer 14 that is associated with the lapse of time. Examples of the moisturizer include polyhydric alcohols such as glycerin, sorbitol, ethylene glycol, propylene glycol, polyethylene glycol, 1,3-propanediol, and 1,4-butanediol, and also include hyaluronic acid, collagen, ceramide, urea, and heparin. One of these moisturizers may be used singly, or two or more may be used in combination. The content of the moisturizer may be regulated to 0.1 to 70% by mass or 3 to 70% by mass, with respect to the total mass of the adhesive layer 14.

Due to the polyhydric alcohol contained in the adhesive layer 14, there is a tendency that the adhesive layer 14 is softer and the adhesion of the microneedle patch 10 is more improved.

In a case where glycerin is contained in the adhesive layer 14 as a polyhydric alcohol, the content of glycerin may be 5 to 70% by mass, may be 10 to 50% by mass, or may be 15 to 45% by mass, with respect to the total mass of the adhesive layer 14. In a case where the content of glycerin is 5% by mass or more, drying of the adhesive layer 14 can be more retarded during the use of the microneedle patch 10, thus making it easy to maintain the adhesion strength of the microneedle patch 10 for a long period of time. In a case where the content of glycerin is 50% by mass or less, glycerin is unlikely to be dissociated from the adhesive layer 14, and the surface of the adhesive layer 14 is more unlikely to be sticky.

Examples of the cooling agent include thymol, l-menthol, dl-menthol, l-isopulegol, and mentha oil. As the cooling agent, l-menthol may be used. The content of the cooling agent may be regulated to 0.5 to 3% by mass, with respect to the total mass of the adhesive layer 14.

Examples of the stabilizer include oxybenzone, dibutylhydroxytoluene (BHT), sodium edetate, and UV absorbers (such as dibenzoylmethane derivatives). The content of the stabilizer may be regulated to 0.01 to 3% by mass, with respect to the total mass of the adhesive layer 14.

Examples of the filler include calcium carbonate, magnesium carbonate, silicate salts (such as aluminum silicate and magnesium silicate), silicic acids, barium sulfate, calcium sulfate, calcium plumbite, zinc oxide, and titanium oxide. The content of the filler may be 0.01 to 10% by mass or 3 to 9% by mass, with respect to the total mass of the adhesive layer 14.

Examples of the preservative include methyl parahydroxybenzoate, ethyl parahydroxybenzoate, propyl parahydroxybenzoate, and butyl parahydroxybenzoate.

Examples of the chelating agent include ethylenediaminetetraacetate, pyrophosphate, hexamethaphosphate, and gluconic acid. The content of the chelating agent may be 0.01 to 1% by mass, 0.05 to 0.5% by mass, or 0.1 to 0.3% by mass, with respect to the total mass of the adhesive layer 14.

Examples of the pH adjuster include organic acids such as acetic acid, lactic acid, oxalic acid, citric acid, and tartaric acid; inorganic acids such as hydrochloric acid, sulfuric acid, nitric acid, and phosphoric acid; and pharmaceutically acceptable salts of the organic acids and the inorganic acids. The content of the pH adjuster may be 0.01 to 1% by mass, 0.05 to 0.7% by mass, or 0.1 to 0.5% by mass, with respect to the total mass of the adhesive layer 14.

As the inorganic powder, colorant, and flavoring, the compositions that are commonly used for the microneedle patch 10 may be used.

For example, the active ingredient may be selected from the group consisting of antioxidants, free radical scavengers, moisturizers, depigmentation agents, lipid controlling agents, ultraviolet reflective agents, wetting agents, anti-microbial agents, allergy prevention drugs, anti-acne drugs, anti-aging drugs, wrinkle prevention drug, fungicides, analgesics, cough medicines, anti-itch drugs, topical anesthetics, hair loss prevention agents, hair growth assistant agents, hair growth suppressors, anti-dandruff agents, anti-histamine agents, keratolytic drugs, anti-inflammatory drugs, soft drinks, therapeutic drugs, anti-infective drugs, inflammation prevention agents, antiemetics, anticholinergics, vasopressors, vasodilators, external wound healing aids, peptides, polypeptides, protein, deodorants, anti-perspirants, emollients, skin moisturizers, softeners, hair conditioners, hair softeners, hair moisturizers, tanners, whitening agents, antifungal agents, depilation agents, external analgesics, counterirritants, hemorrhoidal drugs, pesticides, therapeutic drugs for poison ivy, therapeutic drugs for poison oak, therapeutic drugs for burn, anti-diaper rash drugs, heat rash drugs, skin lotions, vitamins, amino acids, amino acid derivatives, herb extracts, retinoid, flavonoid, sensory markers, skin conditioners, hair lighteners, chelating agents, cell turnover enhancers, coloring agents, sunscreen agents, anesthetic drugs, immunomodulators, nourishing drugs, moisture absorbents, sebum absorbing agents, skin beautifying ingredients, and mixtures thereof.

The active ingredient may contain, for example, a plant preparation such as extract or tincture, for the treatment of a topical skin disease. Examples of the extract or tincture include extraction of oak bark, extraction of walnut, tincture of arnica, extract of witch hazel, extract of ribwort plantain, extract of pansy, extract of thyme or sage; tincture of St. John's wort, extract of golden glow, extract of chamomile flower, or tincture of pot marigold; extract of leaves of birch, extract of nettle, extract of coltsfoot, tincture of comfrey, extract of field horsetail, or extract of aloe for the care of seriously tired and scratched skin; extract of horse chestnut and butcher's broom, and extract of arnica, pot marigold, and chili pepper.

As the active ingredient, the amino acid may include not only salts, esters, or acyl derivatives of amino acids, but also include amino acids obtained by hydrolysis of various proteins. Examples of such amino acid chemicals include amphoteric amino acids such as alkylamide alkylamine, stearyl acetyl glutamate, capryloyl silk amino acid, and capryloyl collagen amino acid; capryloyl keratin amino acid; capryloyl pea amino acid; cocodimonium hydroxypropyl amino acid silk; corn gluten amino acid; cysteine; glutamic acid; glycine; hair keratin amino acid; hair amino acids such as aspartic acid, threonine, serine, glutamic acid, proline, glycine, alanine, half cystine, valine, methionine, isoleucine, leucine, tyrosine, phenylalanine, cysteine acid, lysine, histidine, arginine, cysteine, tryptophan, and citrulline; lysine; silk amino acid; wheat amino acid; and mixtures thereof.

As the active ingredient, peptide, polypeptide, and protein include, for example, a long chain having at least about 10 carbon atoms, and for example, a polymer having a high molecular weight of at least 1000, and they are formed by self-condensation of amino acids. Examples of such a protein include collagen; deoxyribonuclease; iodinated corn protein; keratin; milk protein; protease; serum protein; silk; sweet almond protein; wheat malt protein; wheat protein; α and β helices of wheat protein and keratin protein; and hair proteins such as intermediate filament protein, high sulfur content protein, significantly high sulfur content protein, intermediate filament-related protein, high tyrosine protein, high glycine/tyrosine protein, trichohyalin, and mixtures thereof.

Examples of anti-wrinkle ingredients include hyaluronic acid, sodium hyaluronate, retinol (vitamin A), silybin peptides (HTC collagen, palmitoyl penta, peptide 3, and argireline), amino acids, hydroxyproline, tocopheryl retinoate, ursolic acid, vitamin C derivatives, coenzyme Q10, astaxanthin, fullerene, polyphenols, α lipoic acid, soybean extract, pullulan, activated isoflavone, saccharides, polysaccharides, glycerin, and glycerin derivatives. The anti-wrinkle ingredient may be a mixture of at least two of these compositions.

Examples of the vitamin include vitamin B complexes; vitamins A, C, D, E, and K and derivatives thereof including thiamine, nicotinic acid, biotin, pantothenic acid, choline, riboflavin, vitamin B6, vitamin B12, pyridoxine, inositol, and carnitine, such as vitamin A palmitate; and provitamins such as panthenol (provitamin B5) and panthenol triacetate; and mixtures thereof.

Examples of antibacterial substances include bacitracin, erythromycin, neomycin, tetracycline, chlortetracycline, benzethonium chloride, phenol, and mixtures thereof.

Examples of the emollient and skin moisturizer include mineral oil, lanolin, plant oil, isostearyl isostearate, glyceryl laurate, methyl gluceth-10, methyl gluceth-20, chitosan, and mixtures thereof.

Examples of the hair conditioner include not only lipophilic compounds such as cetyl alcohol, stearyl alcohol, hydrogenated polydecene, and mixtures thereof, but also quaternary compounds such as behenamidopropyl PG-dimonium chloride, tricetyl ammonium chloride, hydrogenated tallow amidoethyl hydroxyethylmonium methosulfate, and mixtures thereof.

Examples of the sunscreen agent include butyl methoxydibenzoylmethane, octyl methoxycinnamate, oxybenzone, octocrylene, octyl salicylate, phenylbenzimidazole sulfonic acid, ethylhydroxypropyl aminobenzoate, menthyl anthranilate, aminobenzoic acid, cinoxate, diethanol amine methoxycinnamate, glycerin aminobenzoate, titanium dioxide, zinc oxide, oxybenzone, Padimate O, red vaseline, and mixtures thereof. Examples of the tanner include dihydroxyacetone.

Examples of skin whitening agents include hydroquinone and derivatives thereof, catechol and derivatives thereof, ascorbic acid and derivatives thereof, ellagic acid and derivatives thereof, kojic acid and derivatives thereof, tranexamic acid and derivatives thereof, resorcinol derivatives, placenta extract, arbutin, oil-soluble licorice root extract, and mixtures thereof.

Examples of anti-inflammatory analgesic drugs include acetaminophen, methyl salicylate, salicylic acid mono glycol, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, and tiaramide hydrochloride. Examples of steroidal anti-inflammatory analgesic drugs that may be used with the microneedle patch 10 include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumethasone, fluorometholone, and beclomethasone dipropionate.

Examples of anti-histamines include diphenhydramine hydrochloride, diphenhydramine salicylate, diphenhydramine, chlorpheniramine hydrochloride, chlorpheniramine maleate, isothipendyl hydrochloride, tripelennamine hydrochloride, promethazine hydrochloride, and methdilazine hydrochloride. Examples of the topical anaesthetic that may be used with the microneedle patch 10 include dibucaine hydrochloride, dibucaine, lidocaine hydrochloride, lidocaine, benzocaine, p-butylaminobenzoic acid 2-(diethylamino)ethyl hydrochloride, procaine hydrochloride, tetracaine, tetracaine hydrochloride, chloroprocaine hydrochloride, oxybuprocaine hydrochloride, mepivacaine, cocaine hydrochloride, piperocaine hydrochloride, dyclonine, and dyclonine hydrochloride.

Examples of bactericides and disinfectants include thimerosal, phenol, isopropyl methylphenol, thymol, benzalkonium chloride, benzethonium chloride, chlorhexidine, povidone iodine, cetylpyridinium chloride, eugenol, and trimethyl ammonium bromide. Examples of the vasopressor that may be used with the microneedle patch 10 include naphazoline nitrate, tetrahydrozoline hydrochloride, oxymetazoline hydrochloride, phenylephrine hydrochloride, and tramazoline hydrochloride. Examples of hemostatics that may be used with the microneedle patch 10 include thrombin, phytonadione, protamine sulfate, aminocaproic acid, tranexamic acid, carbazochrome, carbazochrome sodium sulfonate, rutin, and hesperidin.

Examples of chemotherapeutic drugs include sulfamine, sulfathiazole, sulfadiazine, homosulfamine, sulfisoxazole, sulfisomidine, sulfamethizole, and nitrofurazone. Examples of antibiotics that may be used with the microneedle patch 10 include penicillin, methicillin, oxacillin, cephalothin, cephalosin, erythromycin, lincomycin, tetracycline, chlorotetracycline, oxytetracycline, methacycline, chloramphenicol, kanamycin, streptomycin, gentamicin, bacitracin, and cycloserine.

Examples of antiviral drugs include protease inhibitors, thymidine kinase inhibitors, saccharide or glycoprotein synthesis inhibitors, constituent protein synthesis inhibitors, adhesion and adsorption inhibitors, and nucleoside analogs such as aciclovir, penciclovir, valacyclovir, and ganciclovir.

Examples of hair regrowth or hair growth drugs include minoxidil, carpronium chloride, pentadecanoic acid glyceride, tocopherol acetate, piroctone olamine, glycyrrhizic acid, hinokitiol, swertia herb extraction, ceramide and precursors, nicotinamide, and chili pepper tincture.

Examples of cosmetic active ingredients include D-α-tocopherol, DL-α-tocopherol, D-α-tocopheryl acetate, DL-α-tocopheryl acetate, ascorbyl palmitate, vitamin D, vitamin D2, vitamin D3, retinol, retinol ester, retinyl palmitate, retinyl propionate, β-carotene, coenzyme Q10, D-panthenol, farnesol, and farnesyl acetate; jojoba oil and black currant oil abundantly contained in essential fatty acids; 5-n-octanoylsalicylic acid and esters thereof, salicylic acid and esters thereof; alkyl esters of α-hydroxy acids such as citric acid, lactic acid, and glycolic acid; asiatic acid, madecassic acid, asiaticoside, total extract of Centella asiatica, β-glycyrrhetic acid, α-bisabolol, ceramides such as 2-oleoylamino-1,3-octadecane; phytantriol, marine-derived phosphatides abundantly contained in polyunsaturated essential fatty acids, ethoxyquin; extract of rosemary, extract of balm, quercetin, extract of dried microalgae, anti-inflammatory drugs such as steroidal anti-inflammatory drugs, and biochemical stimulants such as compounds obtained by synthesis of hormones or fats and/or proteins.

Vitamin C promotes collagen (connective tissue) synthesis, metabolism of lipids (fats) and carbohydrates, and synthesis of neurotransmitters. Vitamin C contributes to the optimal maintenance of the immune system. Vitamin C is harmful to a wide variety of cancer cells, in particular, melanoma. The activity of tyrosine oxidase, which catalyzes the aerobic activity of tyrosine that changes into melanin and other pigments, is also prevented by the presence of vitamin C. Vitamin C is found to be effective in catalyzing the immune response to the infection of a lot of viruses and bacteria. In addition to the above-mentioned many applications, vitamin C is essential for collagen synthesis and trauma care. The microneedle patch 10 may contain vitamin C, vitamin E, and a combination of other ingredients such as a moisturizer, a collagen synthesis promoter, and a facial scrub.

Skin conditioner ingredients may include mineral oil, vaseline, plant oils (e.g., soybean oil or maleated soybean oil), dimethicone, dimethicone copolyol, cationic monomers and polymers (e.g., guar hydroxypropyltrimony chloride and distearyl dimethyl ammonium chloride), and mixtures thereof. The moisturizer may be, for example, polyols such as sorbitol, glycerin, propylene glycol, ethylene glycol, polyethylene glycol, polypropylene glycol, 1,3-butanediol, hexylene glycol, isoprene glycol, xylitol, fructose, and mixtures thereof.

The skin beautifying ingredient may include tea extract (e.g., Karatsu tea extract, Ureshino tea extract, and Shizuoka tea extract), sake cake extract (e.g., junmai daiginjo sake cake extract, daiginjo sake cake extract, junmai ginjo sake cake extract, and ginjo sake cake extract), citrus fruit extract (e.g., genko extract, sudachi extract, and Citrus unshiu extract), seaweed extract (e.g., laver extract), plant extract (e.g., asparagus extract), and mixtures thereof.

These active ingredients may be used singly, or two or more may be used in combination. When these active ingredients are pharmaceutically acceptable salts, active ingredients in any form of inorganic salts or organic salts may be of course included. The active ingredient may be directly coated on the skin, and thereafter, the microneedle array 20 described below may be applied to the same portion of the skin. In this case, the infiltration of the active ingredient into the skin can be promoted by the effect of stretching the skin and the ODT (occlusive dressing technique) effect on the skin.

The mass of the adhesive layer 14 may be, for example, 100 g/m² or more, or may be 250 g/m² or more. The mass of the adhesive layer 14 may be, for example, 2000 g/m² or less, or may be 1500 g/m² or less. The mass of the adhesive layer 14 may be 250 to 1500 g/m², and in this case, the adhesive layer fits well, and moreover, an improved adhesion can be maintained over a longer period of time. By setting the mass of the adhesive layer **14** as above, the whole thickness of the microneedle patch 10 can be reduced, and as a result, the microneedle patch 10 is likely to conform to the skin and is hardly released.

The release sheet 16 is a sheet-shaped member that protects the adhesive layer 14 before use of the microneedle patch 10.

A weakened part 16a may be formed on the release sheet 16 over the entire length or the entire width thereof. The weakened part 16a is provided to make it easy to divide the release sheet 16. Although the weakened part 16a is linear perforation in the example of Figure 1, the aspect of the weakened part 16a is not limited thereto. For example, the weakened part 16a may be thin, half-cut, or in other forms. The weakened part 16a may exhibit a waveform or a sawtooth form.

As the release sheet 16, a plastic film such as polypropylene (e.g., unstretched polypropylene and stretched polypropylene), polyethylene terephthalate, polybutylene terephthalate, polyethylene, polyester, polyurethane, polyvinyl chloride, and polystyrene may be used, synthesis resin, synthesis paper, or silicon processed paper obtained by subjecting synthetic fiber to silicon processing may be used, or laminated paper obtained by laminating aluminum foil or kraft paper may be used. The release sheet 16 may be colorless or at least a part thereof may be colored.

The microneedle array 20 is a tool for needling or pressing the skin. Although the example of Figure 1 shows one microneedle array 20, a plurality of microneedle arrays 20 may be embedded in the adhesive layer 14. In a case where a plurality of microneedle arrays 20 is used, the shape and dimension thereof may be uniform, or at least one of the shape and the dimension may be different from each other.

In one example, at least part of the microneedle array 20 is embedded by the adhesive layer 14. That is, at least part of the microneedle array 20 is positioned within the adhesive layer 14. For example, at least a part of each microneedle 22 may be positioned within the adhesive layer 14. Alternatively, the entire microneedle array 20 may be embedded by the adhesive layer 14, and in this case, the whole of each microneedle 22 is positioned within the adhesive layer 14.

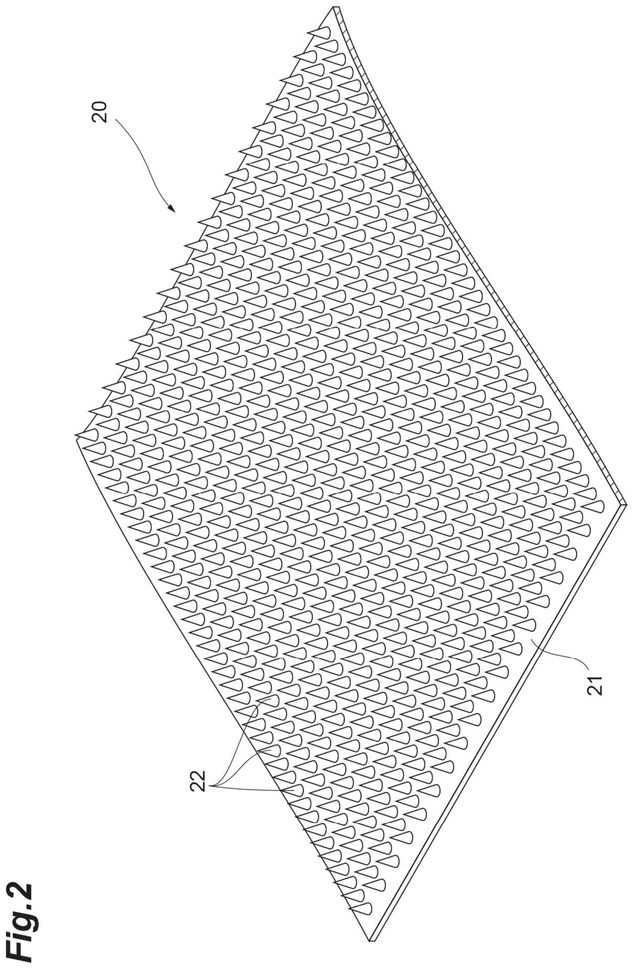

Figure 2 is an enlarged perspective view showing an example of the microneedle array 20. In one example, the microneedle array 20 includes a base 21 and at least one microneedle 22 provided on the base 21.

As the material for the microneedle array 20 (the base 21 and the microneedle 22), silicon, silicon dioxide, ceramic, a metal (such as stainless, titanium, nickel, molybdenum, chrome, and cobalt), or a synthetic or natural resin material may be used. Alternatively, in consideration of the antigenicity and the unit price of the material of the microneedle 22, a biodegradable polymer such as polylactic acid, polyglycolide, polylactic acid-co-polyglycolide, pullulan, caprolactone, polyurethane, and polyanhydride may be used, or a synthetic or natural resin material which is a non-biodegradable polymer, such as polyethylene, polypropylene, polyamide, polyethylene terephthalate, cyclic olefin copolymers, polycarbonate, polymethacrylic acid, polymethyl methacrylate, hard vinyl chloride, ethylene vinyl acetate, polytetrafluoroethylene, tetrafluoroethylene-ethylene copolymers, polyoxymethylene, and acrylonitrile-butadiene-styrene copolymers, may be used. Alternatively, hyaluronic acid, sodium hyaluronate, pullulan, dextran, dextrin or chondroitin sulfate, or a cellulose derivative, which are polysaccharides, may be used. In consideration of probability that the microneedle 22 is broken on the skin, biodegradable resin may be employed, and for example, polylactic acid may be used. Polylactic acid includes polylactic acid homopolymers such as poly L-lactic acid and poly D-lactic acid, poly L/D-lactic acid copolymers, and mixtures thereof, and any of these may be used. The higher the average molecular weight of polylactic acid, the higher the strength thereof, and for example, a polylactic acid having an average molecular weight of 40000 to 100000 may be used. The base 21 and the microneedle 22 may be produced from the same material, or may be produced from materials different from each other.

The base 21 is a basis for supporting the microneedle 22. In the present disclosure, the surface on which the microneedle 22 stands refers to the main surface of the base 21, and the opposite side of the main surface refers to the rear surface of the base 21. In the example of Figure 1, the rear surface of the base 21 is in contact with the main surface of the backing 12. The dimension of the base 21 may be determined in consideration of the dimension of the adhesive layer 14. In the example of Figure 1, the area of the base 21 is smaller than the area of the adhesive layer 14. In another example, the base 21 and the adhesive layer 14 may have the same area. Alternatively, the base 21 may have a larger area than the area of the adhesive layer 14, and in this case, a part of the base 21 is exposed. The area of the base 21 may be 0.5 cm² to 300 cm², may be 1 cm² to 100 cm², or may be 1 cm² to 50 cm². Alternatively, the area of the base 21 may be 0.5 cm² to 10 cm², 0.5 cm² to 5 cm², 1 cm² to 5 cm², 0.5 cm² to 3 cm², or 1 cm² to 3 cm². By connecting some bases 21, a base having a desired size may be formed. The lower limit of the thickness of the base 21 may be 5 µm or may be 20 µm, and the upper limit of the thickness thereof may be 1000 µm or may be 300 µm. The thickness of the base 21 may be 0.5 to 2.5 times the length of the microneedles 22 described below.

The microneedle 22 is a fine structure standing from the main surface of the base 21. The microneedle 22 exhibits a tapered shape that becomes narrower from the bottom being in contact with the base 21 towards the tip portion. The term "microneedle" in this specification is a concept including not only a needle-shaped structure in a broad sense and a structure including a needle shape, but also a shape in which the tip is not sharp.

The lower limit of the density of the microneedle 22 may be, for example, 0.05 needles/cm², 1 needle/cm², 50 needles/cm², 75 needles/cm², 100 needles/cm², 150 needles/cm², 200 needles/cm², 300 needles/cm², 340 needles/cm², or 400 needles/cm². On the other hand, the upper limit of the density may be, for example, 10000 needles/cm², 5000 needles/cm², 2000 needles/cm², 1200 needles/cm², 700 needles/cm², 640 needles/cm², or 340 needles/cm². The lower limit of the density is a value obtained by converting the number of microneedles capable of administering 1 mg of an active ingredient and a required area. The upper limit of the density is the limit value in consideration of the shape of the microneedles. In one example, the lower limit and upper limit of the density of the microneedles 22 are set in consideration of stretching the skin by the microneedles 22 to increase the area of the adhesive layer 14 in contact with the skin.

The length of the microneedle 22 may be 150 to 1200 µm, 200 to 1000 µm, or 300 to 900 µm. The length of the microneedle 22 refers to the distance from the bottom being in contact with the base 21 to the tip. Setting the length of the microneedle 22 to 300 µm or more is to ensure the percutaneous absorption of the active ingredient. By setting the length of the microneedle 22 to 900 µm or less, the microneedle 22 is prevented from being brought into contact with nerves and the probability of causing pain is reduced, and moreover, the probability of bleeding can be prevented. **In** a case where the length of the microneedle is 500 µm or less, the amount of the active ingredient that should be taken intradermally can be efficiently administered, and for example, it is possible to administer the active ingredient without perforating the basement membrane. In one example, the length of the microneedle 22 is selected so that the stratum corneum of skin is not penetrated in normal use, but some microneedles 22 may penetrate through the stratum corneum. That is, the epidermis is stretched by the microneedle 22 and becomes thinner, and the active ingredient penetrates into the epidermis which has become more penetrable, but part of the active ingredient may also enter into the skin through holes formed in the corneum.

In the example of Figure 2, the microneedle 22 is a three-dimensional shape. For example, the microneedle 22 may be a circular cone or any pyramid such as a quadrangular pyramid. The microneedle 22 may not be a cone, and for example, the tip portion may be flat or rounded. The flat or rounded tip portion is obtained by intentional processing in some cases, or resultantly obtained without such processing in some cases. In a case where the tip portion is flat, the area of the flat portion may be 20 to 600 µm² or may be 50 to 250 µm². In a case where the tip portion is rounded, the radius of curvature of the tip portion may be 2 to 100 µm, or 5 to 30 µm.

Examples of the method for producing the microneedle array 20 having the microneedle 22 in a three-dimensional shape include wet etching or dry etching using a silicon base, precise machining using metal or resin (such as electrical discharge machining, laser processing, dicing processing, hot emboss processing, and injection molding processing), and mechanical cutting. By these processing methods, the base 21 and the microneedle 22 are integrally formed. Examples of the method for making the microneedle 22 to have a hollow include a method in which the microneedle 22 is produced and then subjected to secondary processing such as laser processing.

The active ingredient may be contained in the microneedle 22, or a coating containing the active ingredient may be formed on the surface of the microneedle 22.

The microneedle 22 may have a planar shape. For example, the microneedle 22 may be a triangle, or may have other shapes such as rhombus. The microneedle 22 having a planar shape is made by punching the base 21 to form a microneedle 22, and raising the microneedle 22 from the base 21. Therefore, the base 21 and the microneedle 22 have the same thickness. In a case where the base 21 is metal, the microneedle array 20 can be formed by punching the sheet with a chemical solution to form a number of microneedles 22 and raising the microneedles 22. In a case where the base 21 is nonmetal, one method may be to punch the base 21 by a laser to form a number of microneedles 22 and raise the microneedles 22, as in the case of the metal sheet. In a case where etching is used as above, gaps are generated around each microneedle 22.

Regardless of whether the microneedle 22 has a three-dimensional shape or a planar shape, the direction which the tip of the microneedle 22 points (the tip direction of the microneedle 22) may be perpendicular to the main surface of the base 21, or may be the sharp angle direction to the main surface (that is, the tilt angle of the microneedle 22 is larger than 0° and less than 90°).

Then, one example of the production method of the microneedle patch 10 will be described. First, an adhesive composition is spread on a release sheet 16 to form an adhesive layer 14. Subsequently, a microneedle array 20 is arranged on the adhesive layer 14 such that at least a part of each microneedle 22 is embedded in the adhesive layer 14. Subsequently, a backing 12 is placed on the adhesive layer 14 and the microneedle array 20 such that the main surface of the backing 12 and the rear surface of a base 21 are brought into contact with each other. As a result, the adhesive layer 14 and the microneedle 22 are sandwiched between the backing 12 and the release sheet 16.

The microneedle patch 10 may be stored inside a packaging bag. Examples of the material of the packaging bag include aluminum. With such a storage method, a decrease of the water content of the adhesive layer 14 can be suppressed, and attachment of foreign matter and the like to the adhesive layer 14 can be reduced. The microneedle patch 10 may be stored in the packaging bag in a multiply folded state. In this case, since the space required for the storage of the microneedle patch 10 is saved, the material used for the packaging bag can be reduced.

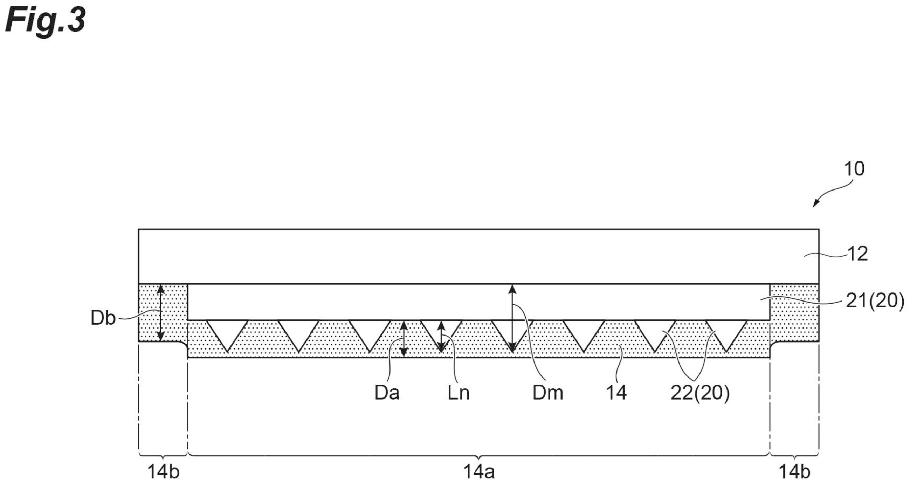

In the microneedle patch 10 before being applied to the skin, the entire microneedle 22 may be embedded within the adhesive layer 14, or the tip of the microneedle 22 may protrude from the adhesive layer 14. The relationship between the adhesive layer 14 and the microneedle 22 will be described with reference to Figure 3. Figure 3 is a diagram schematically showing the microneedle patch 10 viewed from the side.

The adhesive layer 14 includes a main portion 14a that is a region positioned on the microneedle array 20. In a case where the microneedle array 20 is arranged on part of the main surface of the backing 12, the adhesive layer 14 further includes, in addition to the main portion 14a, a peripheral portion 14b that is a region positioned outside the microneedle array 20. It may be said that the peripheral portion 14b is a region where the microneedle array 20 does not exist. The adhesive layer 14 shown in Figures 1 and 3 includes the main portion 14a and the peripheral portion 14b. In a case where a plurality of microneedle arrays 20 are arranged apart from each other on the backing 12, at least part of the peripheral portion 14b may be positioned inside the microneedle patch 10. In the example of Figure 3, the action surface of the peripheral portion 14b is recessed compared to the action surface of the main portion 14a, but the action surface of the adhesive layer 14 may be flat or substantially flat as a whole.

The relationship between the adhesive layer 14 and the microneedle 22 may be represented by the thickness of the adhesive layer 14 and the length of the microneedle 22. The thickness of the adhesive layer 14 refers to the length of the adhesive layer 14 along the direction from the main surface of the backing 12 toward the action surface of the adhesive layer 14. The thickness of the adhesive layer 14 may be the thickness of the main portion 14a or the peripheral portion 14b. It may be said that the thickness of the main portion 14a is the distance from the main surface of the base 21 to the action surface of the adhesive layer 14. It may be said that the thickness of the peripheral portion 14b is the distance from the main surface of the backing 12 to the action surface of the adhesive layer 14. In one example, the thickness of the main portion 14a (of the peripheral portion 14b) may be calculated as a statistical value of the thickness at each of a plurality of measurement points in the main portion 14a (in the peripheral portion 14b). The statistical value may be an average value.

Before the microneedle patch 10 is applied to the skin, the thickness Da of the main portion 14a may be 0.9 to 1.3 times the length Ln of the microneedle 22, or may be 0.9 to 1.2 times the length Ln. The fact that the ratio is less than 1.0 means that the tip of the microneedle 22 protrudes from the adhesive layer 14. The fact that the ratio is greater than 1.0 means that the entire microneedle 22 is embedded within the adhesive layer 14.

In one example, the thickness Db of the peripheral portion 14b may be smaller than the thickness Dm of the microneedle array 20. The thickness of the microneedle array 20 refers to the distance from the rear surface of the base 21 to the tip of the microneedle 22.

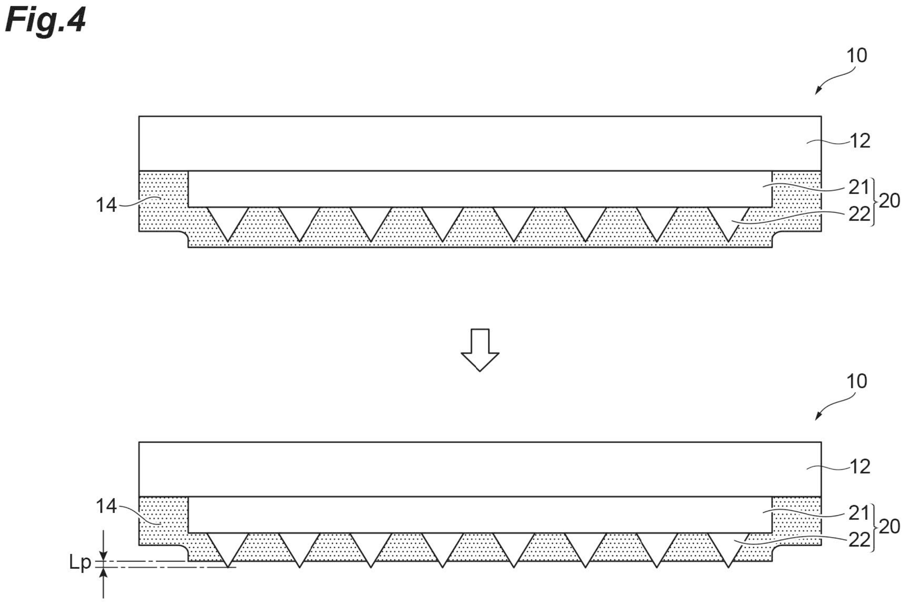

Figure 4 is a diagram schematically showing changes in the microneedle patch 10 before and after application to the skin. It is noted that in this figure, the microneedle patch 10 "deemed to be applied" to the skin is shown as the microneedle patch 10 applied to the skin. After the microneedle patch 10 is applied to the skin, water in the adhesive layer 14 evaporates and the adhesive layer 14 gradually becomes thinner, so the tip of the microneedle 22 protrudes from the adhesive layer 14, or if the tip was already protruding before the application, it protrudes further. In the present disclosure, the length Lp of the portion of the microneedle 22 protruding from the adhesive layer 14 is also referred to as "protruding length". The protruding length is greater after a predetermined time has elapsed from application of the microneedle patch 10 to the skin than before the microneedle patch 10 is applied to the skin. In one example, the predetermined time may be a time selected from 1 minute to 8 hours, and may be, for example, 2 hours, 4 hours, 6 hours, or 8 hours. For example, the protruding length after 8 hours have elapsed from application of the microneedle patch 10 to the skin may be 10 µm or more grater, 20 µm or more greater, or may be 28 µm or more greater than the protruding length before the microneedle patch 10 is applied to the skin.

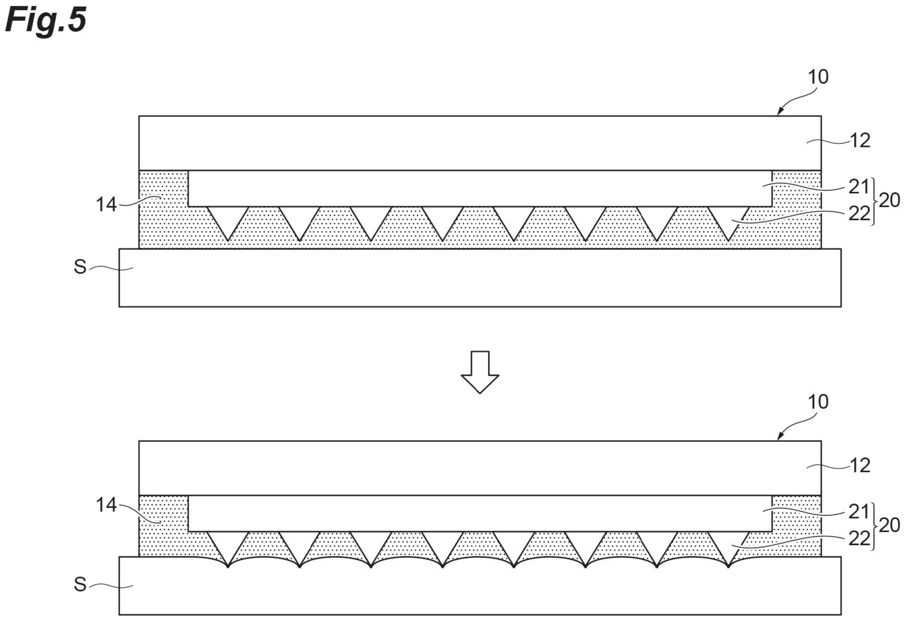

Figure 5 is a diagram schematically showing application of the microneedle patch 10 to skin S. As described above, after the microneedle patch 10 is applied to the skin S, the tip of the microneedle 22 protrudes from the adhesive layer 14, or if the tip was already protruding before application, it protrudes further. The skin S is stretched by the protruding microneedle 22, and the adhesive layer 14 enters into the stretched portion, so the contact area between the skin S and the adhesive layer 14 increases. As a result, the permeability of the active ingredient through the skin S may be improved. Also, since the microneedle 22 does not protrude excessively from the adhesive layer 14, that is, since the protruding length is suppressed, sufficient adhesion of the adhesive layer 14 to the skin S may be maintained.

Hereinabove, the present disclosure has been described in detail based on the embodiments. However, the present disclosure is not limited to the above examples. Various modifications can be made within a range not departing from the gist of the present disclosure.

In the above example, the microneedle patch 10 exhibits a substantially rectangular shape, but the microneedle patch may have other shapes. For example, the microneedle patch may have any shape such as a square, a circle, an oval, a crescent, a comma shape, or other polygonal shapes. In the above example, the shape of the microneedle array 20 is substantially rectangular, but the microneedle array may have other shapes. For example, the microneedle array may have any shape such as a square, a circle, an oval, a crescent, or other polygonal shapes. The shape of the microneedle array may be the same or different from the shape of the microneedle patch.

### [Appendix]

As understood from the various examples described above, the present disclosure includes the following aspects.

### (Appendix 1)

A microneedle patch, comprising:
a backing;
an adhesive layer provided on a main surface of the backing; and
a microneedle array, at least part of the microneedle array being positioned within the adhesive layer, wherein
the adhesive layer comprises a water-soluble polymer, glycerin, and water,
a content of the water is 45 to 70% by mass with respect to a total mass of the adhesive layer,
the microneedle array comprises a base and at least one microneedle standing from a main surface of the base,
the adhesive layer comprises a main portion positioned on the microneedle array, and
a thickness of the main portion is 0.9 to 1.3 times a length of the microneedle.

### (Appendix 2)

The microneedle patch according to appendix 1, wherein the length of the microneedle is 300 to 900 µm.

### (Appendix 3)

The microneedle patch according to appendix 1 or 2, wherein the adhesive layer further comprises a peripheral portion positioned outside the microneedle array.

### (Appendix 4)

The microneedle patch according to appendix 3, wherein a thickness of the peripheral portion is smaller than a thickness of the microneedle array.

### (Appendix 5)

The microneedle patch according to any one of appendices 1 to 4, wherein a protruding length that is a length of a portion of the microneedle protruding from the adhesive layer is greater after a predetermined time has elapsed from application of the microneedle patch to skin than before the microneedle patch is applied to the skin.

### (Appendix 6)

The microneedle patch according to appendix 5, wherein the protruding length after 8 hours have elapsed from the application of the microneedle patch to the skin is 10 µm or more greater than the protruding length before the microneedle patch is applied to the skin.

According to appendix 1, each of the water content and the ratio of the thickness of the adhesive layer on the microneedle array to the length of the microneedle is set within the corresponding numerical range described above. With this configuration, while adhesion of the microneedle patch to the skin is maintained, the skin is stretched by the microneedle protruding from the adhesive layer. As a result, the contact area between the stretched skin and the adhesive layer increases, so that transdermal administration of the active ingredient may be efficiently performed. In another aspect, the microneedle protruding from the adhesive layer come into contact with the skin, thereby improving the adhesion of the microneedle patch to the skin during application.

According to appendix 2, the length of the microneedle is set within the above numerical range. With this configuration, while adhesion of the microneedle patch to the skin is more reliably maintained, the skin is stretched by the microneedle protruding from the adhesive layer. As a result, the contact area between the stretched skin and the adhesive layer increases, so that transdermal administration of the active ingredient may be efficiently performed.

According to appendix 3, since the adhesive layer is also positioned outside the microneedle array, the degree of reduction of the adhesive layer after the microneedle patch is applied to the skin may be more easily controlled. For example, the phenomenon of microneedle protruding excessively from the adhesive layer may be suppressed more reliably and sufficient adhesion of the adhesive layer to the skin may be maintained.

According to appendix 4, since the thickness of the adhesive layer positioned outside the microneedle array is limited, the microneedle may be more reliably protruded from the adhesive layer, and the skin may be more reliably stretched by the microneedle.

According to appendix 5, the protruding length of the microneedle gradually increases with application of the microneedle patch to the skin. Therefore, the contact area between the skin and the adhesive layer may be increased while gradually stretching the skin.

According to appendix 6, the protruding length of the microneedle gradually increases over 8 hours with application of the microneedle patch to the skin. Therefore, during the application time, the contact area between the skin and the adhesive layer may be increased while gradually stretching the skin.

### Examples

Hereinafter, examples will be specifically described, but the present disclosure is not limited thereto.

### [Thickness of adhesive layer (1)]

According to the composition (unit: % by mass) shown in Table 1, an adhesive composition for the microneedle patch according to Example 1 was prepared. Using the adhesive composition, one microneedle patch was produced by the production method described above. As for the microneedle array, microneedles having a length of 475 µm were provided on a substantially circular base having a thickness of 700 µm and an area of 1 cm², at a density of 156 pieces/cm².

**[Table 1]**

| Composition | Example 1 |
|---|---|
| Neutralized polyacrylate | 7.7 |
| Polyvinyl alcohol | 4.1 |
| Sodium carboxymethylcellulose | 1.8 |
| Tartaric acid | 0.3 |
| Alum | 0.28 |
| Disodium ethylenediaminetetraacetate | 0.2 |
| Isopropyl methylphenol | 0.1 |
| Others | 1.2 |
| Glycerin | 19.62 |
| Purified water | 64.7 |
| Total | 100.00 |

Thereafter, the protruding length of the microneedles was calculated at each time point of 0 hours, 2 hours, 4 hours, 6 hours, and 8 hours as shown below.
(1) In the first step, using a CO₂ laser marker LP-420, which is a laser cutter manufactured by Panasonic Device SUNX Co., Ltd., the produced microneedle patch was cut along the thickness direction of the microneedle array so that a cross-section of the microneedle array was formed.
(2) In the second step, using a microscope VHX-2000 manufactured by Keyence Corporation, a cross-sectional image of the cut surface was acquired at a magnification of 100 times. Then, based on the cross-sectional image, the thickness was measured at 9 positions of the main portion of the adhesive layer, and the average value of the measured thicknesses was obtained as the thickness of the main portion at the 0-hour time point. It may be said that this thickness is a thickness before applying the microneedle patch to skin.
(3) In the third step, the microneedle patch having the cut surface was left in a stationary state for 2 hours in a space with an environmental temperature of 24±3°C and a relative humidity of 50±10% RH.
(4) In the fourth step, using the laser cutter, the microneedle patch was cut parallel to the cut surface in the first step, at the portion where the microneedle array existed.
(5) In the fifth step, similarly to the second step, the thickness of the main portion of the adhesive layer at the 2-hour time point was calculated based on the cross-sectional image of the new cut surface. It may be said that this thickness is a thickness after applying the microneedle patch to skin.
(6) In the sixth step, the protruding length at the 2-hour time point was obtained by subtracting the thickness obtained in the fifth step from the length of the microneedles. In addition, the ratio of the thickness of the main portion of the adhesive layer to the length of the microneedles was calculated.
(7) In the seventh step, the third to sixth steps were performed for each of 4-hour, 6-hour, and 8-hour time points to obtain the protruding length and ratio at each time point.

Table 2 shows the results obtained by the above processing. The "Thickness of adhesive layer" is the thickness of the main portion of the adhesive layer. The "Thickness/length ratio" is the ratio of the thickness of the main portion of the adhesive layer to the length of the microneedles. At the 0-hour time point, since the thickness of the adhesive layer was greater than the length of the microneedles, the protruding length was 0 µm, and the thickness/length ratio was greater than 1. After 2 hours had elapsed from the start of application of the microneedle patch, the microneedles protruded from the adhesive layer, and the thickness/length ratio became less than 1. It was also found that the thickness of the adhesive layer tended to decrease linearly. The coefficient of determination R² in linear regression was 0.9521.

**[Table 2]**

| Example 1 | | | |
|---|---|---|---|
| Time | Thickness of adhesive layer (µm) | Protruding length (µm) | Thickness/length ratio |
| Before application (0 hours) | 492 | 0 | 1.04 |
| 2 hours later | 420 | 55 | 0.88 |
| 4 hours later | 418 | 57 | 0.88 |
| 6 hours later | 338 | 137 | 0.71 |
| 8 hours later | 280 | 195 | 0.59 |

### [Thickness of adhesive layer (2)]

According to the compositions (unit: % by mass) shown in Table 3, adhesive compositions for the microneedle patches according to Examples 2 to 12 and Comparative Examples 1 to 3, respectively, were prepared. Using these adhesive compositions, three microneedle patches were produced in each example and each comparative example by the production method described above. As for the microneedle array, microneedles of selected lengths were provided on a substantially circular base having a thickness of 700 µm and an area of 1 cm². The density of the microneedles was 1180 pieces/cm² in Example 5, 316 pieces/cm² in Examples 7 and 8, and 156 pieces/cm² in the other examples and comparative examples.

**[Table 3]**

| Composition | Comparative Example 1 | Example 2 | Examples 3, 5-12 Comparative Examples 2, 3 | Example 4 |
|---|---|---|---|---|
| Neutralized polyacrylate | 6.5 | 6.5 | 6.5 | 6.5 |
| Polyvinyl alcohol | 3.0 | 3.0 | 3.0 | 3.0 |
| Sodium carboxymethylcellulose | 2.0 | 2.0 | 2.0 | 2.0 |
| Tartaric acid | 0.3 | 0.3 | 0.3 | 0.3 |
| Alum | 0.25 | 0.25 | 0.25 | 0.25 |
| Disodium ethylenediaminetetraacetate | 0.2 | 0.2 | 0.2 | 0.2 |
| Isopropyl methylphenol | 0.1 | 0.1 | 0.1 | 0.1 |
| Others | 1.0 | 1.0 | 1.0 | 1.0 |
| Glycerin | 46.65 | 41.65 | 34.65 | 16.65 |
| Purified water | 40.0 | 45.0 | 52.0 | 70.0 |
| Total | 100.00 | 100.00 | 100.00 | 100.00 |

Furthermore, according to the compositions (unit: % by mass) shown in Table 4, adhesive compositions for the microneedle patches according to Examples 12A, 12B, 12C, and 12D, respectively, were prepared. Using these adhesive compositions, three microneedle patches were produced in each of these four examples by the production method described above. As for the microneedle array, microneedles having a length of 475 µm were provided on a substantially circular base having a thickness of 700 µm and an area of 1 cm². In these four examples, the density of the microneedles was 156 pieces/cm².

**[Table 4]**

| Composition | Example 12A | Example 12B | Example 12C | Example 12D |
|---|---|---|---|---|
| Neutralized polyacrylate | 6.5 | 6.5 | 11.5 | 6.5 |
| Polyvinyl alcohol | 3.0 | 3.0 | - | 3.0 |
| Sodium carboxymethylcellulose | 2.0 | 2.0 | - | 2.0 |
| Tartaric acid | - | 0.3 | 0.3 | 0.3 |
| Alum | 0.25 | - | 0.25 | 0.25 |
| Magnesium aluminometasilicate | - | 0.25 | - | - |
| Disodium ethylenediaminetetraacetate | 0.2 | 0.2 | 0.2 | 0.2 |
| Isopropyl methylphenol | 0.1 | 0.1 | 0.1 | 0.1 |
| Others | 1.0 | 1.0 | 1.0 | 1.0 |
| Glycerin | 34.65 | 34.65 | 34.65 | 34.65 |
| Purified water | 52.3 | 52.0 | 52.0 | 52.0 |
| Total | 100.00 | 100.00 | 100.00 | 100.00 |

Thereafter, for each example and each comparative example shown in Tables 3 and 4, the protruding length was calculated at each time point of 0 hours and 8 hours as shown below.
(1) In the first step, using the above laser cutter, each of the three microneedle patches was cut along the thickness direction of the microneedle array so that a cross-section of the microneedle array was formed.
(2) In the second step, using the above microscope, cross-sectional images of the cut surfaces were acquired at a magnification of 100 times for each of the three microneedle patches. Subsequently, for each microneedle patch, the thickness was measured at 9 positions of the main portion of the adhesive layer based on the cross-sectional image. Then, the average value of the measured thicknesses of the three microneedle patches was obtained as the thickness of the main portion at the 0-hour time point, that is, the thickness before application. Also, the thickness/length ratio was calculated using that thickness. Furthermore, for each microneedle patch, the thickness was measured at 9 positions of the peripheral portion of the adhesive layer based on the cross-sectional image. Then, the average value of the measured thicknesses of the three microneedle patches was obtained as the thickness of the peripheral portion at the 0-hour time point.
(3) In the third step, the microneedle patches having the cut surfaces were left in a stationary state for 8 hours in a space with an environmental temperature of 24±3°C and a relative humidity of 50±10% RH.
(4) In the fourth step, using the laser cutter, each of the three microneedle patches was cut parallel to the cut surface in the first step, at the portion where the microneedle array existed.
(5) In the fifth step, similarly to the second step, the thickness of the main portion of the adhesive layer at the 8-hour time point was calculated based on the cross-sectional image of the new cut surface. It may be said that this thickness is the thickness after applying the microneedle patch to skin.
(6) In the sixth step, for each of the three microneedle patches, the protruding length at the 8-hour time point was calculated by subtracting the thickness of the adhesive layer from the length of the microneedles. The thickness of the adhesive layer used in this calculation was not the average value obtained in the same manner as the second step in the fifth step, but was the average value of nine measured thicknesses in one microneedle patch for which the protruding length was to be calculated. Then, the average value of the obtained three protruding lengths was obtained as the protruding length at the 8-hour time point. Also, for each of the second step and the fifth step, the thickness/length ratio was calculated from the thickness of the main portion of the adhesive layer obtained in the step and the length of the microneedles.

Table 5 shows the results of Comparative Example 1 and Examples 2 to 4 obtained by the above processing. The meanings of "thickness of adhesive layer" and "thickness/length ratio" are the same as in Table 2. In a case where the water content was 45 to 70% by mass, the microneedles protruded from the adhesive layer during the application of the microneedle patch.

**[Table 5]**

| | | Comparative Example 1 | Example 2 | Example 3 | Example 4 |
|---|---|---|---|---|---|
| Time | Purified water (% by mass) | 40.0 | 45.0 | 52.0 | 70.0 |
| | Length of Microneedles (µm) | 475 | 475 | 475 | 475 |
| After 0 hours | Thickness of adhesive layer (µm) | 490 | 499 | 488 | 439 |
| | Protruding length (µm) | 0 | 0 | 0 | 36 |
| | Thickness/length ratio | 1.03 | 1.05 | 1.03 | 0.92 |
| After 8 hours | Thickness of adhesive layer (µm) | 482 | 449 | 380 | 303 |
| | Protruding length (µm) | 4 | 28 | 95 | 172 |
| | Thickness/length ratio | 1.01 | 0.95 | 0.80 | 0.64 |

Table 6 shows the results of Examples 5 to 8 obtained by the above processing. The meanings of "thickness of adhesive layer" and "thickness/length ratio" are the same as in Table 2. In a case where the length of the microneedles was 300 to 900 µm, the microneedles protruded from the adhesive layer or the protruding length increased, during the application of the microneedle patch.

**[Table 6]**

| | | Example 5 | Example 6 | Example 7 | Example 8 |
|---|---|---|---|---|---|
| Time | Length of Microneedles (µm) | 300 | 475 | 700 | 900 |
| After 0 hours | Thickness of adhesive layer (µm) | 301 | 460 | 666 | 834 |
| | Protruding length (µm) | 8 | 15 | 34 | 66 |
| | Thickness/length ratio | 1.00 | 0.97 | 0.95 | 0.93 |
| After 8 hours | Thickness of adhesive layer (µm) | 235 | 368 | 533 | 703 |
| | Protruding length (µm) | 65 | 107 | 167 | 197 |
| | Thickness/length ratio | 0.78 | 0.77 | 0.76 | 0.78 |

Table 7 shows the results of Examples 3, 6, 9 to 12, 12A, 12B, 12C, 12D, and Comparative Examples 2 and 3 obtained by the above processing. In this table, the thicknesses of the main portion and the peripheral portion are shown as the thickness of the adhesive layer. The meaning of "thickness/length ratio" is the same as in Table 2. In a case where the thickness of the main portion of the adhesive layer was 0.9 to 1.3 times the length of the microneedles, the microneedles protruded from the adhesive layer or the protruding length increased, during the application of the microneedle patch.

**[Table 7]**

| | | Example 6 | Example 9 | Example 10 | Example 3 | Example 11 | Example 12 | Example 12A | Example 12B | Example 12C | Example 12D | Comparative Example 2 | Comparative Example 3 |
|---|---|---|---|---|---|---|---|---|---|---|---|---|---|
| Time | Length of Microneedles (µm) | 475 | 475 | 475 | 475 | 475 | 475 | 475 | 475 | 475 | 475 | 475 | 475 |
| After 0 hours | Thickness of main portion of adhesive layer (µm) | 460 | 476 | 483 | 488 | 506 | 571 | 548 | 503 | 535 | 567 | 695 | 1030 |
| | Thickness of peripheral portion of adhesive layer (µm) | 451 | 377 | 544 | 922 | 1031 | 1359 | 1419 | 1452 | 1492 | 1336 | 1330 | 1880 |
| | Protruding length (µm) | 15 | 1 | 0 | 0 | 4 | 0 | 0 | 0 | 0 | 0 | 0 | 0 |
| | Thickness/length ratio for main portion | 0.97 | 1.00 | 1.02 | 1.03 | 1.06 | 1.20 | 1.15 | 1.06 | 1.13 | 1.19 | 1.46 | 2.17 |
| After 8 hours | Thickness of main portion of adhesive layer (µm) | 368 | 394 | 384 | 380 | 422 | 482 | 464 | 420 | 445 | 437 | 619 | 968 |
| | Protruding length (µm) | 107 | 81 | 91 | 95 | 62 | 34 | 11 | 55 | 31 | 38 | 0 | 0 |
| | Thickness/length ratio for main portion | 0.77 | 0.83 | 0.81 | 0.80 | 0.89 | 1.01 | 0.98 | 0.88 | 0.94 | 0.92 | 1.30 | 2.04 |

### [Probe tack test]

According to the compositions (unit: % by mass) shown in Table 8, adhesive compositions for the microneedle patches according to Examples 13 and 14 and Comparative Examples 4 and 5, respectively, were prepared. Using these adhesive compositions, three microneedle patches were produced in each example and each comparative example by the production method described above. As for the microneedle array, microneedles having a length of 475 µm were provided on a substantially circular base having a thickness of 700 µm and an area of 1 cm², at a density of 156 pieces/cm². Regarding Example 14 and Comparative Examples 4 and 5, the products were left at room temperature to volatilize water in the adhesive layer, thereby making the adhesive layer thinner, and thus microneedle patches according to Example 14 and Comparative Examples 4 and 5, respectively, were obtained. The composition of the adhesive layer during production was adjusted such that the composition of the adhesive layer during the probe tack test would be the same among Examples 13 and 14 and Comparative Examples 4 and 5.

The meanings of "thickness of adhesive layer" and "thickness/length ratio" in Table 8 are the same as in Table 2.

**[Table 8]**

| Composition | Comparative Example 4 | | Comparative Example 5 | | Example 13 | | Example 14 |
|---|---|---|---|---|---|---|---|
| | At prod. | At test | At prod. | At test | At prod. | At test | At prod. and test |
| Neutralized polyacrylate | 7.0 | 9.4 | 7.7 | 9.4 | 8.5 | 9.4 | 9.4 |
| Polyvinyl alcohol | 3.8 | 5.1 | 4.1 | 5.1 | 4.6 | 5.1 | 5.1 |
| Sodium carboxymethylcellulose | 1.6 | 2.2 | 1.8 | 2.2 | 2.0 | 2.2 | 2.2 |
| Tartaric acid | 0.3 | 0.4 | 0.3 | 0.4 | 0.3 | 0.4 | 0.4 |
| Alum | 0.26 | 0.35 | 0.28 | 0.35 | 0.31 | 0.35 | 0.35 |
| Disodium ethylenediaminetetraacetate | 0.2 | 0.3 | 0.2 | 0.3 | 0.3 | 0.3 | 0.3 |
| Isopropyl methylphenol | 0.1 | 0.1 | 0.1 | 0.1 | 0.1 | 0.1 | 0.1 |
| Others | 1.1 | 1.5 | 1.2 | 1.5 | 1.3 | 1.5 | 1.5 |
| Glycerin | 18.24 | 24.15 | 19.62 | 24.15 | 21.59 | 24.15 | 24.15 |
| Purified water | 67.4 | 56.5 | 64.7 | 56.5 | 61.0 | 56.5 | 56.5 |
| Total | 100.00 | 100.00 | 100.00 | 100.00 | 100.00 | 100.00 | 100.00 |
| Length of Microneedles (µm) | 475 | 475 | 475 | 475 | 475 | 475 | 475 |
| Thickness of adhesive layer (µm) | - | 327 | - | 370 | - | 444 | 486 |
| Thickness/length ratio | - | 0.69 | - | 0.78 | - | 0.93 | 1.02 |

Furthermore, according to the composition (unit: % by mass) shown in Table 9, an adhesive composition for the microneedle patch according to Example 13A was prepared. Using this adhesive composition, three microneedle patches were produced also in Example 13A by the production method described above. As for the microneedle array, microneedles having a length of 475 µm were provided on a substantially circular base having a thickness of 700 µm and an area of 1 cm², at a density of 156 pieces/cm².

The meanings of "thickness of adhesive layer" and "thickness/length ratio" in Table 9 are the same as in Table 2.

**[Table 9]**

| Composition | Example 13A |
|---|---|
| Neutralized polyacrylate | 9.4 |
| Polyvinyl alcohol | 5.1 |
| Sodium carboxymethylcellulose | 2.2 |
| Tartaric acid | 0.4 |
| Magnesium aluminometasilicate | 0.35 |
| Disodium ethylenediaminetetraacetate | 0.3 |
| Isopropyl methylphenol | 0.1 |
| Others | 1.5 |
| Glycerin | 24.15 |
| Purified water | 56.5 |
| Total | 100.00 |
| Length of Microneedles (µm) | 475 |
| Thickness of adhesive layer (µm) | 431 |
| Thickness/length ratio | 0.91 |

Using TA.XT plus, which is a texture analyzer manufactured by Stable Micro Systems, the adhesive force was measured for each of Examples 13, 13A, and 14 and Comparative Examples 4 and 5 as follows.
(1) In the first step, human skin excised at a thickness of about 500 µm using a dermatome and cut into a circular shape with a diameter of 10 mm was fixed to the tip of a circular probe with a diameter of 10 mm with the epidermal side facing outward. This work was performed for each of three samples.
(2) In the second step, the microneedle patch was fixed to the stage of the texture analyzer with the adhesive layer facing upward. The position of the microneedle patch on the stage was adjusted such that the excised human skin fixed to the probe was in contact with the main portion of the adhesive layer. This work was performed for each of three samples.
(3) In the third step, the probe was lowered at a speed of 0.2 mm/sec to bring the human skin into contact with the microneedle patch, and a load of 5 g was applied for 1 second. Subsequently, the probe was pulled up at a speed of 10 mm/sec, and the adhesive force (unit: g·sec) was measured by integrating the load from the start of pulling up until the probe separated from the adhesive layer. It may be said that the measured value is the adhesive force of the main portion of the adhesive layer. This measurement was performed for each of three samples, and the average value of the adhesive force was obtained.

The average values of the adhesive force were as follows, and Examples 13, 13A, and 14 were superior in adhesion to Comparative Examples 4 and 5.
- Comparative Example 4: 0.250 g·sec
- Comparative Example 5: 0.492 g·sec
- Example 13: 0.987 g·sec
- Example 13A: 1.214 g·sec
- Example 14: 0.975 g·sec

### [Density of microneedles]

Adhesive compositions for the microneedle patches according to Examples 15 to 17 were prepared with the same compositions as Examples 3 and 5 to 12 described above. Using these adhesive compositions, one microneedle patch was produced in each Example by the production method described above. As for the microneedle array, microneedles having a length of 475 µm were provided at selected densities on a substantially circular base having a thickness of 700 µm and an area of 1 cm².

Then, for each Example, the protruding length was calculated at each time point of 0 hours and 8 hours in the same manner as Examples 3 and 5 to 12.

Table 10 shows the results of Examples 3 and 15 to 17. The meanings of "thickness of adhesive layer" and "thickness/length ratio" are the same as in Table 2. In all cases, the microneedles protruded from the adhesive layer during the application of the microneedle patch.

**[Table 10]**

| | | Example 15 | Example 3 | Example 16 | Example 17 |
|---|---|---|---|---|---|
| Time | Density of microneedles (needles/cm²) | 76 | 156 | 340 | 640 |
| After 0 hours | Thickness of adhesive layer (µm) | 505 | 488 | 528 | 506 |
| | Protruding length (µm) | 0 | 0 | 0 | 3 |
| | Thickness/length ratio | 1.06 | 1.03 | 1.11 | 1.07 |
| After 8 hours | Thickness of adhesive layer (µm) | 364 | 380 | 385 | 400 |
| | Protruding length (µm) | 111 | 95 | 90 | 75 |
| | Thickness/length ratio | 0.77 | 0.80 | 0.81 | 0.84 |

### [Stretching of skin by microneedles]

According to the composition (unit: % by mass) shown in Table 11, an adhesive composition for the microneedle patch according to Example 18 was prepared.

**[Table 11]**

| Composition | Example 18 |
|---|---|
| Neutralized polyacrylate | 6.5 |
| Polyvinyl alcohol | 3.0 |
| Sodium carboxymethylcellulose | 2.0 |
| Tartaric acid | 0.3 |
| Alum | 0.25 |
| Disodium ethylenediaminetetraacetate | 0.2 |
| Isopropyl methylphenol | 0.1 |
| Others | 1.0 |
| Zirconium oxide | 1.0 |
| Glycerin | 15.65 |
| Purified water | 70.0 |
| Total | 100.00 |

Then, as shown below, the microneedle patch according to Example 18 was produced, and the length of the skin surface in contact with the adhesive layer was calculated at each time point of 0 hours and 8 hours.
(1) **In** the first step, for imaging by X-ray CT, the surface of the microneedle array was gold-coated using IB-3, which is an ion coater manufactured by Eiko Co., Ltd. As for the microneedle array, microneedles having a length of 475 µm were provided on a substantially circular base having a thickness of 700 µm and an area of 1 cm², at a density of 156 pieces/cm².
(2) **In** the second step, for imaging by X-ray CT, one microneedle patch was produced using the above adhesive composition to which zirconium oxide was added as a contrast agent. The thickness of the main portion of the adhesive layer before application was 439 µm, and therefore, the thickness/length ratio was 0.92.
(3) **In** the third step, each of polystyrene foam and excised human skin was prepared with dimensions of 20 mm × 20 mm.
(4) In the fourth step, the microneedle patch was adhered to the epidermis side of the excised human skin to prepare a sample.
(5) In the fifth step, the sample was placed on the polystyrene foam with the side opposite to the epidermis of the excised human skin facing downward, and after fixing the sample to the polystyrene foam using tape, the sample was measured with TDM1000H-II (2K), which is an X-ray CT apparatus manufactured by Yamato Scientific Co., Ltd. This measurement result was acquired as the result at the 0-hour time point.
(6) In the sixth step, another sample obtained in the fourth step was left in a stationary state for 8 hours in a space with an environmental temperature of 24±3°C and a relative humidity of 50±10% RH. This sample was also measured in the same manner as the fifth step, and this measurement result was acquired as the result at the 8-hour time point.
(7) For each of the 0-hour time point and the 8-hour time point, the length of the skin surface in a section of 7 mm in linear distance was analyzed from the measurement result. In each sample, the length of the skin surface was measured in each of four different sections, and the average value of the measured values was obtained as the final length.

Furthermore, the same adhesive composition as Example 18, that is, the adhesive composition shown in Table 11, was prepared for Examples 18A, 18B, and 18C. Then, microneedle patches according to Examples 18A, 18B, and 18C were produced by the same production method as Example 18. The parameters that differed among Examples 18, 18A, 18B, and 18C were the density of the microneedles, the thickness of the main portion of the adhesive layer before application, and the thickness/length ratio. In the same manner as Example 18, for Examples 18A, 18B, and 18C, the length of the skin surface in contact with the adhesive layer was calculated at each time point of 0 hours and 8 hours.

Table 12 shows the results of Examples 18, 18A, 18B, and 18C. These results indicate that the application of the microneedle patch to the skin caused the skin to be stretched, and the area of the adhesive layer in contact with the skin increased.

**[Table 12]**

| Composition | Example 18A | Example 18 | Example 18B | Example 18C |
|---|---|---|---|---|
| Density of microneedles (needles/cm²) | 76 | 156 | 340 | 640 |
| Length of Microneedles (µm) | 475 | 475 | 475 | 475 |
| Thickness of main portion of adhesive layer (µm) | 474 | 439 | 523 | 532 |
| Thickness/length ratio for main portion | 1.00 | 0.92 | 1.10 | 1.12 |
| (A) Length of skin surface at 0 hours (mm) | 7.22 | 7.41 | 7.30 | 7.25 |
| (B) Length of skin surface at 8 hours (mm) | 7.83 | 8.11 | 8.00 | 7.65 |
| Length of stretched skin (B-A) (mm) | 0.61 | 0.70 | 0.70 | 0.40 |

### Reference Signs List

10 ... microneedle patch, 12 ... backing, 14 ... adhesive layer, 14a ... main portion of adhesive layer, 14b ... peripheral portion of adhesive layer, 16 ... release sheet, 16a ... weakened part, 20 ... microneedle array, 21 ... base, 22 ... microneedles.

## Claims

1. A microneedle patch, comprising:
a backing;
an adhesive layer provided on a main surface of the backing; and
a microneedle array, at least part of the microneedle array being positioned within the adhesive layer, wherein
the adhesive layer comprises a water-soluble polymer, glycerin, and water,
a content of the water is 45 to 70% by mass with respect to a total mass of the adhesive layer,
the microneedle array comprises a base and at least one microneedle standing from a main surface of the base,
the adhesive layer comprises a main portion positioned on the microneedle array, and
a thickness of the main portion is 0.9 to 1.3 times a length of the microneedle.

2. The microneedle patch according to claim 1, wherein the length of the microneedle is 300 to 900 µm.

3. The microneedle patch according to claim 1 or 2, wherein the adhesive layer further comprises a peripheral portion positioned outside the microneedle array.

4. The microneedle patch according to claim 3, wherein a thickness of the peripheral portion is smaller than a thickness of the microneedle array.

5. The microneedle patch according to claim 1 or 2, wherein a protruding length that is a length of a portion of the microneedle protruding from the adhesive layer is greater after a predetermined time has elapsed from application of the microneedle patch to skin than before the microneedle patch is applied to the skin.

6. The microneedle patch according to claim 5, wherein the protruding length after 8 hours have elapsed from the application of the microneedle patch to the skin is 10 µm or more greater than the protruding length before the microneedle patch is applied to the skin.
